# EUROPEAN PATENT SPECIFICATION

(11) **EP 0 738 114 B1**
(45) Date of publication and mention of the grant of the patent: **05.09.2001**
(21) Application number: 94905819.2
(22) Date of filing: 06.01.1994
(51) Int. Cl.: A23L 1/015, A23L 2/00

(54) **FOOD PRODUCTS FOR PREVENTING DEVELOPMENT OF DISEASES AND PROCESS FOR PREPARING SAME**
NAHRUNGSMITTEL ZUR VERHINDERUNG DER ENTWICKLUNG VON ERKRANKUNGEN UND VERFAHREN ZUR HERSTELLUNG DIESER NAHRUNGSMITTEL
PRODUITS ALIMENTAIRES EMPECHANT L'APPARITION DE MALADIES, ET LEUR PROCEDE DE PREPARATION

(43) Date of publication of application: 23.10.1996
(73) Proprietor: HYD KUTATO-FEJLESZTO KTF., 1215 Budapest (HU); Somlyai, Gábor, 1215 Budapest (HU)
(72) Inventor: SOMLYAI, Gábor, H-1215 Budapest (HU)
(74) Representative: Beszédes, Stephan G., Dr.
(86) International application number: HU9400001
(87) International publication number: WO9518545

(56) References cited:
- DE-A- 4 232 465

## Description

The invention relates to food products which prevent the development of diseases. Furthermore, the invention relates to a process for preparing these products.

It is known that in human organism from the moment of conception up to the date of death 10¹⁶ cell divisions take place. The cell division is preceded by the doubling of genetic information, DNA. The enzymes responsible for the DNA synthesis copy with 10⁻⁶ probability one code with defect, resulting in a gene which is different from the original one (mutant). In most cases that "mistake" creates no problem for the cells. However, when these mutations occur on certain points of the genes which play a central role in the regulation of cell division, as a consequence of the change in the genetic code shortly yet another doubling of the defective cell occurs and the process continues whereby a group of cell develops which outgrow neighbouring cells. If the organism does not recognize in time the rapidly multiplying cells, the process can lead to the development of a malignant tumour.

It takes years for a tumour to develop. According to current test methods it takes 4 bis 5 years for one malignant cell to develop into a tumour of 1 cm diameter, for one cell to multiply into one hundred million. During that period malignant cells have enough time to scatter all through the organism and start developing other tumours as well. The reasons for the modest results achieved in the struggle against tumours include the fact that by the time the tumour is diagnosed in a body the malignant cells may be present throughout the organism.

For a long time the defense mechanism of the organism is able to maintain a balance with the continuously emerging tumour cells.

The probability for the balance to turn over is growing with the age of a person. The balance may be turned up and the tumour may develop due to a long-lasting disease which keeps the immune system engaged, or a long-term stress situation may also make it easier for the damaging effects of the so-called civilised society (smoking, nitrate-containing drinking water, polluted air, etc.) to take their toll. People who are affected with all the above effects are more likely to become victim of malignant tumour.

On the other hand, there are effects which are unfavourable for the development of tumours. For instance it has been observed that a mother of three children is less likely to develop breast tumour than a mother of two children, or one with a single child or a women who is childless. That is explained by the fact that the hormone effects accompanying three pregnancies reduce the chance for the development of malignant breast tumour.

These observations draw attention to the possibility of developing conditions which are unfavourable for tumour cells carrying mutation, and thus they can be eliminated. By cure-like treatment it is possible for the human organism to defend itself from tumour development before the tumour reaches a size in which it can already be detected.

Furthermore in DE-A1-4 232 465 there has been disclosed the use of products made out of deuterium-depleted water having a deuterium concentration of 0.1 to 110 ppm for prevent cancer.

The problem underlying to the present invention is to prepare food products which make it possible to prevent the development of tumoral diseases by eliminating tumours of as yet undetectable size through changes taking place in the organism.

The invention starts from the recognition that, depending on the climate, the D concentration of water containing 140 to 155 ppm deuterium or of plants grown with the use of water of such D content is a precondition for the maintenance of normal cell multiplication. It has been rerecognized that the D concentration relative to H concentration as an element of a sub-molecular system initiates cell division through the increase of D concentration in relation to that of H. The D entering the organism is especially indispensable for the rapidly multiplying tumour cells.

The basis of the invention is the recognition that D-depleted water, solutions and foods in a definite range of D concentrations higher than the D concentrations used in DE-A1-4 232 465 can stop the division of tumour cells by reducing the D content of the organism in an optimum way, and thus they can also prevent the development of tumours and can cure tumoral diseases in an optimum way.

Based on the above the invention is the use of water having a D-concentration between 115 to 135 ppm for preparing food products for preventing development of tumoral diseases.

A subject-matter of the invention is also a process for preparing the food products from water having a D-concentration between 115 to 135 ppm, characterized by carrying out standard electrolysis and/or distillation methods and using the water thus obtained
for the production of soft drinks and beer using standard production processes
   or
as a nutrient solution in algae production or for irrigation of plants used in the food industry for preparing algae and plants with lower D-content, and working up them in the usual way to food products.

The products prepared by the use and process according to the invention are well suited to prevent the development of tumours. The reason is that the consumption of beverages and plants produced with D-depleted water within the determined range will reduce the D content of the organism in an optimum way, slowing down the multiplication of tumoral cells which finally die off.

Experiments carried out with animals fed with D-depleted water prove the suitability of the invention.

PC-3 marked human prostate tumour was implanted in 28 CBA/Ca marked immune-suppressed mice. The treatment of 14 mice with water of 115 ppm D content was started on day 18 after transplantation, by giving the mice such water to drink. The treatment lasted 16 days, then the mice were killed and the tumours were histologically investigated.

The speed of growth of tumoral cells was determined by the ratio of dividing and dead cells in the tumour. Such ratio is easy to determine on properly prepared histological excisions.

Monitoring the growth rate of the tumour on the test animals it could be seen that the group that was fed with D-depleted water had on the average smaller tumours than the control animals. Taking into consideration the obvious fact that the size of the tumour in the animals also depended on the size of the implanted tumours, the effect of D-depleted water was more objectively reflected by histological tests which showed that in the control group the ratio between dividing (mitotic) and dead (apoptose) cells was 3.6 : 1, as against 1.5 : 3 observed with animals fed with D-depleted water according to the definition by the invention.

Solutions with curative effect of the food industry are produced from raw materials normally used for the manufacture of fruit juices, syrups, soft drinks and beer by mixing the active agent with these raw materials. The processing of algae, vegetables and fruits grown with the use of the D-depleted water is also performed by standard methods.

It is favourable to consume food products of the invention every 1 to 2 years for 1 to 3 weeks each time. During the cure it is suitable to consume exclusively plants produced with the use of the D-depleted water.

The main advantages of the invention are as follows:
a) It renders possible to intervene in the cell division regulation mechanism through the same mechanism as that by which cell division itself is regulated.
b) It renders possible to prevent the development of tumours by creating unfavourable conditions for the multiplication of tumoral cells whereby the organism becomes able to eliminate the development of future tumours.
c) The compounds used in the process have no toxic side-effects.
d) No harmful waste arises during the producing process.
e) The products are easy to produce.
f) As the active agent is not mutagenic, no mutant cells develop in the course of the treatment, in contrast to most of the cytotoxins used so far that are very mutagenic and often lead to the development of even further tumours.

The invention is further illustrated by the following Examples.

### Example 1

### Production of D-depleted water using electrolysis

15 to 20% aqueous KOH solution is electrolysed with 2 to 5 V voltage direct current, with a cathode separated from the anode. The hydrogen with lower D content developing on the cathode is burned and the thus-developing water vapour is condensed by a distillation system and collected separately. The D concentration of the water thus obtained is 30 to 40 ppm. This water is mixed with normal water of 150 ppm D concentration in such a ratio that D-depleted water with D content between 115 and 135 ppm is obtained.

The product can be directly used for preventing the development of tumoral diseases or as raw material for the production of compounds with lower D content.

### Example 2

### Production of D-depleted water by distillation

Distilled water is boiled at 45 to 50°C in a distilling tower with 30 to 50 plates used for fractional distillation under 50 to 60 mbar pressure. During distillation reflux value is 12 to 13, sludging in the kettle is 10-fold. By using such parameters the D concentration of the heads is between 20 and 30 ppm. By increasing the number of plates the D content of the water can be further reduced to 1 to 10 ppm. By mixing the water thus produced with normal water of 150 ppm D content, water with any D concentration between 115 and 135 ppm can be obtained.

### Example 3

### Production of fruit juices and carbonated soft drinks with lower D content

By mixing distilled water of 20 to 30 ppm D content in adequate proportion with normal water of 150 ppm D content, water with 115 to 135 ppm D content is produced. This water is used in the usual way for producing fruit juices and carbonated soft drinks.

### Example 4

### Production of beer with lower D content

The barley used for malt production is soaked in water with D content between 115 and 135 ppm, then it is spread to form a 5 to 15 cm thick layer and germinated at low (5 to 15°C) temperature and good ventilation. Germinated barley is dried at a temperature between 50 and 75°C, freed from the remnants of the germ-roots and milled. Milled malt is mixed with adequate quantity of water of D concentration between 115 and 135 ppm and the mixture is kept at a temperature of 50 to 75°C, then filtered and boiled with hop. The beer wort is filtered, cooled and inoculated with pre-grown Saccharomyces cerevisiae. The main fermentation takes place at 5 to 6°C for 10 to 14 days, then post-fermentation is carried out for several weeks at 0°C in airtight barrels. Finally the thus produced beer is filtered, bottled and pasteurised. The D content of the beer depends primarily on the D content of the water used in the manufacturing process, and that affects also the D content of ethanol and other components.

### Example 5

### Production of food with high protein and low D content in algae culture

Chlorella vulgaris is cultured by continuously bubbling a gas mixture of 95% by volume of air and 5% by volume of CO₂ through a nutrient liquid of the following composition: 5 g/l of KNO₃; 1.25 g/l of KH₂PO₄; 2.5 g/l of MgSO₄ x 7 H₂O; 3 mg/l of FeSO₄ x 7 H₂O and 1 ml/l of Arnon A-5 solution (0.6 ml/l of 0.4% tartaric acid; 2.86 g/l of H₃BO₃; 1.81 g/l of MnCl₂ x 4 H₂O; 0.08 g/l of CuSO₄ x 5 H₂O; 0.22 g/l of ZnSO₄ x 7 H₂O; 0.21 g/l of Na₂MoO₄ and one drop of H₂SO₄). Water of 115 to 135 ppm D content is used for the preparation of the nutrient liquid. In a 200 1 size vat 1 kg of dry matter can be produced with natural light in a month.

### Example 6

### Production of vegetables and fruits with low D content

Vegetables and fruits are produced in the standard way in all respects, with the only difference, that water with 115 to 135 ppm D content is used for irrigation. During the photosynthesis the plants can build less D into the organic compounds. By consuming plants produced in this way the organism will have significantly lower D concentration, and that will inhibit the division of tumoral cells.

## Claims

1. The use of water having a D-concentration between 115 to 135 ppm for preparing food products for preventing development of tumoral diseases.

2. A process for preparing the food products from water having a D-concentration between 115 to 135 ppm according to claim 1, characterized by carrying out standard electrolysis and/or distillation methods and using the water thus obtained
for the production of soft drinks and beer using standard production processes
or
as a nutrient solution in algae production or for irrigation of plants used in the food industry for preparing algae and plants with lower D-content, and working up them in the usual way to food products.

## Patentansprüche

1. Verwendung von Wasser mit einer D-Konzentration zwischen 115 und 135 ppm für die Herstellung von Lebensmittelprodukten für die Verhinderung von tumoralen Krankheiten.

2. Ein Verfahren für die Herstellung der Lebensmittelprodukten aus Wasser mit D-Konzentration zwischen 115 und 135 ppm nach Anspruch 1, **dadurch gekennzeichnet**, dass man standardartige Elektrolyse- und/oder Destillationsmethoden durchführt und das derart gewonnene Wasser
für die Herstellung von alkoholfreien Getränken und Bier durch standardartige Herstellungsverfahren
oder
als ein Nährlösung in Algenproduktion oder für die Bewässerung von Pflanzen, die in der Lebensmittelindustrie für die Herstellung von Algen und Pflanzen mit niedrigerem D-Gehalt angewendet werden, verwendet, und man diese in der herkömmlichen Weise zu Lebensmittelprodukten aufarbeitet.

## Revendications

1. Utilisation d'une eau à concentration de deutérium située entre 115 et 135 ppm pour préparer des aliments servant à empêcher le développement de maladies tumorales.

2. Procédé pour préparer les aliments à partir d'eau à concentration de deutérium située entre 115 et 135 ppm selon la Revendication 1, **caractérisé en ce que** l'on se sert de méthodes standard d'électrolyse et/ou de distillation et que l'on utilise l'eau ainsi obtenue
pour la production de boissons rafraîchissantes et de bière par des procédés de production standard
ou
à titre de solution nutritive dans la production d'algues ou pour l'irrigation de plantes utilisées dans l'industrie agroalimentaire afin de préparer des algues et des plantes à teneur diminuée en deutérium, et afin de transformer celles-ci selon la manière habituelle en produits alimentaires.
